# EUROPEAN PATENT APPLICATION

(11) **EP 3 805 388 A1**
(43) Date of publication of application: **14.04.2021**
(21) Application number: 19811422.5
(22) Date of filing: 29.05.2019
(51) Int. Cl.: C12N 15/13, C07K 1/22, C07K 16/00, C12N 15/09, C12P 21/08

(54) **METHOD FOR OBTAINING ANTIBODY, METHOD FOR IDENTIFYING ANTIBODY, METHOD FOR PRODUCING ANTIBODY, AND ANTIBODY**

(30) Priority: 30.05.2018 JP 2018103024
(71) Applicant: Cognano, Inc., Kyoto-shi, Kyoto 601-1255 (JP)
(72) Inventor: HIROSE, Shigehisa, Kyoto-shi, Kyoto 601-1255 (JP)
(74) Representative: Mathys & Squire
(86) International application number: PCT/JP2019/021353
(87) International publication number: WO 2019/230823

(57) **Abstract**

A method of logically acquiring an antibody targeting a target substance to deal with classification of cells (such as mammalian cells), class classification of living things, classification of viruses, class classification of molecules (molecules classification such as an activated species and a non-activated species, a modified species and a nonmodified species, a mutated species and a wild species, a holo body and an apo body, a polymer and a monomer, and a complex and a simple substance), and so on is provided. A method of acquiring an antibody that specifically recognizes a target substance is characterized by subtracting an antibody group that recognizes a control substance from an antibody group that recognizes the target substance.

## Description

### TECHNICAL FIELD

The present invention relates to a method of acquiring an antibody, an antibody specifying method, an antibody manufacturing method, and an antibody and, more specifically, to an antibody detection method of specifically detecting a target substance.

### BACKGROUND ART

Conventionally, in relation to establishment of substances that recognize regions relating to molecule activation and molecule inactivation and molecule regions relating to complex formation, various examinations have been made from the viewpoint of molecular targeted drug discovery.

As for molecules that act on and interfere with these target regions, with a low molecular weight compound, it is not very likely to have influence on activation or complex formation of target molecules. Hence, because of molecular sizes and high specificity, examinations have mainly been done on monoclonal antibodies. However, since monoclonal antibodies are accidentally discovered products, a method of establishing a desired monoclonal antibody requires high cost in terms of both time and economy because of a low probability of recognizing a target epitope.

It is known that a special antibody that has no light chains, called as a heavy chain antibody, exists in the serum of an animal of the family Camelidae (a Bactrian camel, a dromedary, a llama, or the like). An antibody fragment formed by the variable domain of a heavy chain antibody is called a VHH antibody, which has the lowest molecular weight as an immunoglobulin fragment that can bind to an antigen.

A VHH antibody can easily be manufactured by, for example, a method using Escherichia coli or the like or a screening method from a library by the cDNA display method, and also has high refolding performance and high resistance to thermal denaturation. For this reason, the VHH antibody has received a great deal of attention as a next-generation antibody from the viewpoint of industrial application (for example, see PTLs 1 and 2).

### CITATION LIST

### PATENT LITERATURE

PTL 1: Japanese Patent Laid-Open No. 2005-520494
PTL 2: Japanese Patent Laid-Open No. 2017-14112

### SUMMARY OF INVENTION

### TECHNICAL PROBLEM

However, a technique of logically acquiring an antibody while aiming at a small target site does not exist yet.

It is an object of the present invention to provide a method of logically acquiring an antibody while aiming at a target substance.

### SOLUTION TO PROBLEM

According to an embodiment of the present invention, there is provided a method of acquiring an antibody that specifically recognizes a target substance, characterized by subtracting an antibody group that recognizes a control substance from an antibody group that recognizes the target substance.

### ADVANTAGEOUS EFFECTS OF INVENTION

According to the present invention, it is possible to easily and efficiently acquire an antibody that specifically detects a target substance.

Other features and advantages of the present invention will be apparent from the following description taken in conjunction with the accompanying drawings. Note that the same reference numerals denote the same or like components throughout the accompanying drawings.

### BRIEF DESCRIPTION OF DRAWINGS

The accompanying drawings, which are incorporated in and constitute a part of the specification, illustrate embodiments of the invention and, together with the description, serve to explain principles of the invention.

Fig. 1 is a view schematically showing an aspect of subtraction according to the present invention;
Fig. 2 is a view showing a result of cell and tissue staining for a tissue slice of a cancer patient by an antibody acquired by a method according to the present invention;
Fig. 3 is a view showing a result of cell and tissue staining for a tissue slice of a cancer patient by a known monoclonal antibody;
Fig. 4 is a view showing a result of cell and tissue staining for Her2 in HEK 293 by an antibody acquired by the method according to the present invention; and
Fig. 5 is a view showing a result of cell and tissue staining for Her2 in HEK 293 by a known monoclonal antibody.

### DESCRIPTION OF EMBODIMENTS

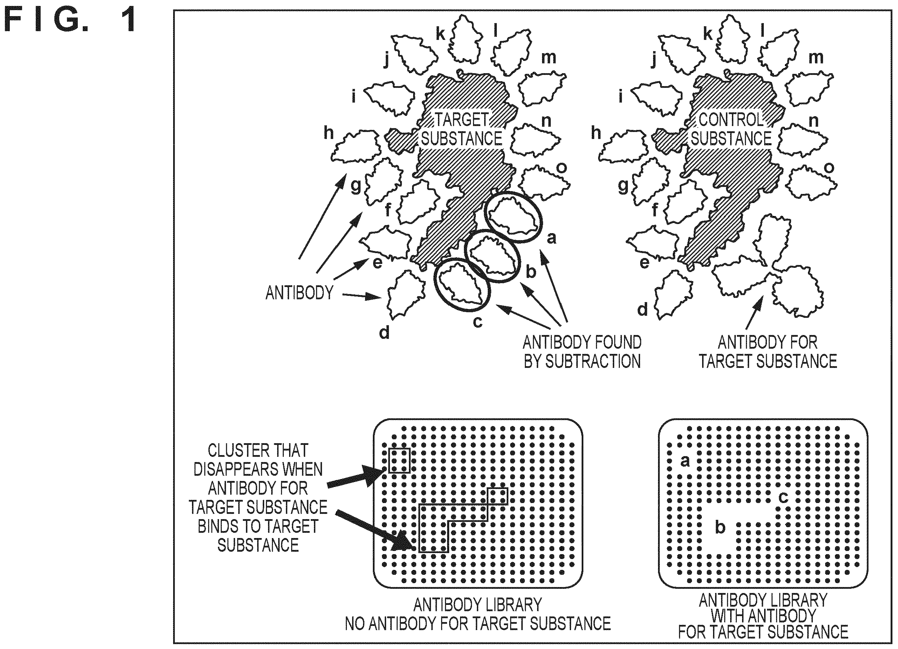

An antibody acquisition method according to the present invention has a feature of subtracting an antibody group that recognizes a control substance from an antibody group that recognizes a target substance. In the present invention, subtraction means that an antibody group binding to a target substance and an antibody group binding to a control substance are compared, thereby finding an antibody (group) that is included in the former but not in the latter. More specifically, for example, as shown in Fig. 1, when antibodies a to o bind to a target substance on the left side, and the antibodies d to o bind to a control substance on the right side, the antibodies a, b, and c can be found by subtraction as antibodies that specifically bind to the target substance. Hence, antibodies suitable for a purpose can efficiently be acquired by combining various target substances and control substances.

In the present invention, the combination of a target substance and a control substance can be set in accordance with a purpose. Detailed examples of purposes are classification of cells, class classification of living things, classification of viruses, and class classification of molecules.

Classification of cells means classifying cell states, developments, types, elapsed times, animal species, differentiations, cycles, gene expressions, presence/absence of fertilization, and the like. Target cells include, for example, mammalian cells, cultured cells, egg cells, germ cells, cancer cells, blood cells, vertebrate cells, invertebrate cells, mononuclear cells, multinucleated cells, prokaryotic cells, eukaryotic cells, living cells, dead cells, and the like. More specifically, examples are classification of cell states (for example, classification of cancer cells and normal cells), classification of cell developments (for example, classification of cells before differentiation and cells after differentiation), classification of cell types (for example, classification of ES cells and iPS cells), classification of times elapsed after cell developments (for example, classification of senescent cells and juvenile cells), classification of animal species of cells (for example, classification of cells of different species such as human and other animals), classification of differentiations with enucleation of cells (for example, classification of cells such as red blood cells before and after enucleation), classification of proliferation cycles of cells (for example, classification of cells in a proliferative phase and in a nonproliferative phase), classification of gene expressions of cells (for example, classification of cells before activation and cells after activation), and classification of fertilized eggs and unfertilized eggs in egg cells (for example, classification of cells before fertilization and cells after fertilization).

Class classification of living things means classifying phylum, class, order, family, genus, species, and the like of living things. Living things include, for example, bacteria, fungi, nematodes, algae, plankton, spiders, insects, fishes, amphibians, reptiles, mammals, and the like. More specifically, examples are classification of Gram-negative bacteria and Gram-positive bacteria, classification of bacterial species such as Escherichia coli and pseudomonas aeruginosa, classification of pathogenic Escherichia coli and nonpathogenic Escherichia coli, classification of fungi such as blue mold and white mold, and classification of nematodes and bug eggs.

Classification of viruses means classifying species, types, and the like of viruses. More specifically, examples are classification of human immunodeficiency viruses, classification of influenza viruses, classification of hepatitis viruses.

Class classification of molecules means classifying conformational states and the like of organic compounds that form living things, such as proteins, sugars, lipids, nucleic acids, and compounds, and organic compounds generated by living things. More specifically, examples are classification of molecules of activated species and non-activated species, classification of molecules of monomers and polymers, classification of molecules of monomers and complexes, classification of molecules of human and other animals, classification of mutated types and wild types (nonmutated types), classification of molecules corresponding to temperatures, classification of molecules at respective pHs, classification of molecules at respective salt concentrations, and classification of molecules in reduced/nonreduced states.

Preferable examples of the combination of a target substance and a control substance in the above-described classifications will be described below.

As the combination of a target substance and a control substance when classifying cells, more specifically, for example, the combinations of an iPS cell and an ES cell, a differentiated cell and a stem cell, a differentiated cell and an undifferentiated cell, a cancer cell and a normal cell, and a senescent cell and a juvenile cell can be used. Both an iPS cell and an ES cell have functions as a stem cell, and the functions are closely resembled. Hence, normally, these can be classified only by genome analysis. However, since different molecules can exist in these cells, an antibody specific to an iPS cell or an antibody specific to an ES cell can be found by subtraction. A differentiated cell and a stem cell have the relationship of a mother cell and a daughter cell from the viewpoint of systematic classification. That is, as a concept, when a stem cell is divided only once, differentiated cells are generated. Since these cells are different only slightly, an antibody specific to a differentiated cell or an antibody specific to a stem cell can be found by subtraction. A differentiated cell and an undifferentiated cell have the relationship of a mother cell and a daughter cell from the viewpoint of systematic classification. An antibody specific to a differentiated cell can be found by subtraction. Both a cancer cell and a normal cell are originated from identical cells. An antibody specific to a cancer cell or an antibody specific to a normal cell can be found by subtraction. A senescent cell and a juvenile cell are identical cells but are different in the time elapsed from development. An antibody specific to a senescent cell or an antibody specific to a juvenile cell can be found by subtraction.

As the combination of a target substance and a control substance when performing class classification of living things, more specifically, for example, the combinations of Escherichia coli and pseudomonas aeruginosa, pathogenic Escherichia coli and nonpathogenic Escherichia coli, an enteric bacterium of human and an enteric bacterium of other animals, and the like can be used. Both Escherichia coli and pseudomonas aeruginosa are classified as bacteria and are different in pathogenicity to human. An antibody that recognizes the difference between the bacterial outer membranes of these can be found by subtraction. Both pathogenic Escherichia coli and nonpathogenic Escherichia coli are classified as Escherichia coli and are different in pathogenicity to human. An antibody that recognizes the difference between the bacterial outer membranes of these can be found by subtraction. Both an enteric bacterium of human and an enteric bacterium of other animals are classified as enteric bacteria and are different in host. An antibody that recognizes the difference between the bacterial outer membranes of these enteric bacteria can be found by subtraction.

As the combination of a target substance and a control substance when classifying viruses, more specifically, for example, the combinations of type A, type B, and type C of human immunodeficiency viruses (AIDS viruses), Zaire type and Sudan type of Ebola viruses, type A, type B, and type C of feline immunodeficiency viruses, type A and type B of human influenza viruses, an avian influenza virus and a swine influenza virus, and the like can be used. Since human immunodeficiency viruses are classified by the type of the envelope surface protein (envelope), an antibody that specifically recognizes the difference between envelopes can be found by subtraction. Since Ebola viruses are classified by the type of the envelope surface protein (envelope), an antibody that specifically recognizes the difference between envelopes can be found by subtraction. Since feline immunodeficiency viruses are classified by the type of the envelope surface protein (envelope), an antibody that specifically recognizes the difference between envelopes can be found by subtraction. Since human influenza viruses are classified by the type of the envelope surface protein (hemagglutinin), an antibody that specifically recognizes the difference between envelopes can be found by subtraction. Since influenza viruses for animals other than human are classified by the type of the envelope surface protein (hemagglutinin), an antibody that specifically recognizes the difference between envelopes can be found by subtraction.

As the combination of a target substance and a control substance when performing class classification of molecules, more specifically, for example, the combinations of an activated species and a non-activated species, a modified species and a nonmodified species, a mutated species and a wild species, a holo body and an apo body, a solvent condition A and a solvent condition B, a monomer and a polymer, and a complex and a simple substance can be used. As the combination of an activated molecule and a normal molecule, for example, an activated molecule to which a ligand binds and a normal molecule to which a ligand does not bind to can be used. An antibody that recognizes the difference between these can be found by subtraction. As the combination of a modified molecule and a nonmodified molecule, for example, a protein with glycosylation and a protein without glycosylation can be used. An antibody that recognizes the difference between these can be found by subtraction. As the combination of a mutated molecule and a wild type molecule, for example, a protein with amino acid residue substitution and a protein with a naturally present amino acid sequence can be used. An antibody that recognizes the difference between these can be found by subtraction. As the combination of a holo body and an apo body of molecules, for example, a holo body in which a ligand binds to a protein and an apo body in which a ligand does not bind to a protein can be used. An antibody that recognizes the difference between these can be found by subtraction. As the combination of the solvent condition A and the solvent condition B, an acidic solvent (solvent condition A) and a basic solvent (solvent condition B) can be used. An antibody that recognizes the difference between molecules under the environments can be found by subtraction. As the combination of a polymer and a monomer of molecules, a dimeric protein and a monomeric protein can be used. An antibody that recognizes the difference between these can be found by subtraction. As the combination of a complex molecule and a simple substance molecule, a complex in which a protein and an antibody bind and a protein simple substance can be used. An antibody that recognizes the difference between these can be found by subtraction.

As described above, the target substance and the control substance can appropriately be set. By combining the target substance and the control substance in a way that only a portion that characterizes the target substance is different and the remaining portions are the same as possible, an antibody suitable for a purpose can be acquired more easily and efficiently.

When an arbitrary antibody is brought into contact with the set target substance and control substance, an antibody group that recognizes the target substance and an antibody group that recognizes the control substance can be obtained. Note that the antibody to be brought into contact may be a known antibody (for example, a commercially available antibody display) or a separately prepared antibody. As a prepared product, an antibody obtained by immunizing an animal or an antibody obtained by preparation in an experimental tool can be used.

For example, when using an antibody obtained by animal immunity, a VHH antibody obtained by immunizing a camel, a dromedary, a llama, an alpaca, or the like is suitably used. VHH antibody acquisition can be performed in accordance with a known method.

The contact condition of the antibody to the target substance or the control substance can appropriately be set based on a substance to be used in accordance with the technical common general knowledge.

The antibody group that recognizes the target substance and the antibody group that recognizes the control substance can thus be acquired. When subtraction of these is performed, an antibody (group) that specifically binds to the target substance can be found. Note that in the subtraction, it is possible to perform subtraction through determination based on the existence of an antibody itself, and the method is not particularly limited. As the method of determining the existence of an antibody, a known method can be used. For example, subtraction may be performed by detecting the difference in the result of a next-generation sequencer. More specifically, antibodies in the antibody group that recognizes the target substance and the antibody group that recognizes the control substance are sequenced by the next-generation sequencer, the existence ratio is determined, and an antibody whose existence ratio is favorably 5:1 to 10:1, or more favorably more than 10:1, can be determined as an antibody that exists specific to the target substance or the control substance and acquired. A numerical value corrected using an internal standard may be used as needed. An antibody to be selected may be processed through clustering. As the conditions of clustering, for example, a group in which only one amino acid residue is different, a group in which only two amino acid residues are different, a group in which only n (n is a natural number of 3 or more) amino acid residues are different, a group in which the number of full-length amino acid residues is the same within the group in which only one amino acid residue is different, a group in which the number of full-length amino acid residues is the same within the group in which only two amino acid residues are different, a group in which the number of full-length amino acid residues is the same within the group in which only n (n is a natural number of 3 or more) amino acid residues are different, and the like can be used. Clustering can be performed before or after subtraction or before and after subtraction. As a detailed aspect, if the number of antibodies to be used is about 1 × 10⁸, it is possible to specify several to tens of antibodies that specifically bind to the target substance and acquire these by the method according to the present invention, although the specific number depends on a substance to be used and cannot unconditionally be defined. Hence, the antibody acquisition method according to the present invention is also an antibody screening method. An example of subtraction will be described below.

For example, a method of acquiring a group of amino acid sequences group (C group, wherein C group = A group - B group) by subtracting a group of amino acid sequences (B group) of a VHH antibody that binds to a protein A under an environment in which a protein B (for example, an antibody) that interacts with the protein A exists from a group of amino acid sequences (A group) of a VHH antibody that interacts with the protein A will be described. First, concerning antibodies in the A group and the B group, for example, in the result of the next-generation sequencer, based on the result of appearance frequency (read count), amino acid sequences that are included in the A group (read count is 2 or more) and are not included in the B group (read count is 0) are selected (Co group); among the amino acid sequences that are included in the A group (read count is 20 or more), amino acid sequences that only exists in the B group by a read count of 1/10 or less thereof (read count is 2 or more) are selected (C_{1/10} group); and among the amino acid sequences that are included in the A group (read count is 4 or more), amino acid sequences that only exists in the B group by a read count of 1/2 or less thereof (read count is 2 or more) are selected (C_{1/2} group). Next, the C group can be defined as a set obtained by adding the amino acid sequences included in the C₀ group, the C_{1/10} group, and the C_{1/2} group and excluding amino acid sequences that duplicate in the three groups. The C group is an antibody group obtained by the antibody acquisition method according to the present invention, and it is also expected that the positive rate in the antibody screening positive rate will be high in the order of the C₀ group, the C_{1/10} group, and the C_{1/2} group.

In this way, it is possible to acquire an antibody that specifically binds to a target substance. The antibody can be obtained by subtracting an antibody group that recognizes a control substance from an antibody group that recognizes a target substance. If the substance that recognizes the target substance or the control substance is a peptide, it can be obtained as a peptide that specifically binds to the target substance. Hence, as an embodiment of the present invention, there is provided a method of acquiring a peptide that specifically binds to a target substance by subtracting a peptide group that recognizes a control substance from a peptide group that recognizes the target substance.

### Examples

The present invention will be described below in detail based on examples, and the present invention is not limited to these.

### Example 1: Induction of Immune Response of Her2 in Alpaca

An immune response in one alpaca by injection of purified Her2 was executed in accordance with a known method. Note that the used Her2 was prepared from tumor cells with Her2 overexpression using a nickel immobilized affinity resin (available from GE healthcare).

### Example 2: Expression and Purification of Her2-His

N-terminus 624 amino acid residues in an extracellular domain of human Her2 and a histidine (His) tag (6xHis) were used. A plasmid expressed 80 kDa Her2-His fusion protein and secreted this in a culture supernatant. Human embryonic kidney cells (HEK 293) were temporarily transfected with Her2-His construct, and the culture supernatant was collected for a further process. An Her2-His fused body was purified from the supernatant using a nickel immobilized column. The function of the Her2-His fused body was evaluated by immunoprecipitation.

Her2-specific mAb (Herceptin) sufficiently binds to the Her2-His fused body in immunoprecipitation. This indicates that the conformation of a binding site of Herceptin on the extracellular domain of the Her2-His fused body is not dramatically affected.

### Example 3: Generation of Herceptin Monoclonal Antibody

Herceptin is a chimera antibody for an anti-Her2 treatment (trastuzumab), which is derived from a mouse monoclonal antibody (Carter P, Presta L, Gorman CM, Ridgway JB, Henner D, Wong WL, Rowland AM, Kotts C, Carver ME, Shepard HM. Humanization of an anti-p185HER2 antibody for human cancer therapy. Proc Natl Acad Sci USA. 1992 May 15;89(10):4285-9.). A Herceptin monoclonal antibody was prepared in accordance with a known method (Anti-erbB-2(Her-2/neu) [4D5-8(trastuzumab)] Absolute antibody).

### Example 4: Binding of Her2 Extracellular Domain to Magnetic Beads

An Her2-His fusion protein produced in the human embryonic kidney cells (HEK 293) was purified from a culture supernatant using a nickel immobilized column. According to conditions recommended by the manufacturer (Invitrogen) of magnetic beads, a primary amino group contained in a protein and an active group (carboxylic acid) existing on the magnetic bead surface were covalently bound using 50mg/mL of EDC (1-ethyl-3-(-3-dimethyl aminopropyl) carbodiimide hydrochloride) and 50mg/mL of NHS (N-hydroxysuccinimide) reagent.

### Example 5: Selection of VHH Antibody That Interacts with Her2

Lymphocytes were collected from the blood of an alpaca in which an immune response was caused by injection of Her2. Acquisition of a group of gene sequences (VHH antibody gene library) of VHH antibodies contained in the lymphocytes and preparation of a group of phages (VHH antibody phage library) having the group of gene sequences were performed by referring to known methods.

A method of binding the group of phages containing the group of gene sequences to magnetic beads with an Her2-His fusion protein was performed under conditions recommended by the manufacturer (Invitrogen) of the magnetic beads. More specifically, 200 µL of magnetic beads with the Her2-His fusion protein were put in a sample tube having a capacity of 1.5 mL. In addition, phages (10,000,000 or more) were added, and stirring was performed for one night, thereby saturating the binding. After the binding to the magnetic beads with the Her2-His fusion protein was saturated, the phages were cleaned using a sufficient amount of buffer (a tris buffer solution containing a surfactant, neutral pH) (1mL, 20-min stirring was performed four times). After the cleaning, the phages bound to the magnetic beads with the Her2-His fusion protein were separated from the magnetic beads with a protease treatment and collected. Escherichia coli (TG1 competent cells) for phage infection was infected with the collected phages and stored as an Escherichia coli library. The group of gene sequences of the VHH antibody bound to the magnetic beads with the Her2-His fusion protein exists in a form of plasmids in the created Escherichia coli library. The plasmid was purified by a method recommended by the manufacturer (Qiagen).

### Example 6: Selection of VHH Antibody That Binds to Her2 under Environment in Which Protein (Herceptin Monoclonal Antibody) That Interacts with Her2 Exists

200 µL of magnetic beads with an Her2-His fusion protein were put in a sample tube having a capacity of 1.5 mL together with 1 µg of Herceptin monoclonal antibody, phages (10,000,000 or more) were added, and stirring was performed for one night, thereby saturating the binding. Then, by the same method as in Example 5, the group of gene sequences of a VHH antibody bound to the magnetic beads of the Her2-His fusion protein to which the Herceptin monoclonal antibody bound was purified in a form of a plasmid.

### Example 7: Determining Gene Sequences of Group of Gene Sequences of VHH Antibody

A gene sequence included in the group of gene sequences of a VHH antibody acquired in each of Examples 5 and 6 was decoded by next-generation sequence analysis. For example, 100,000 types of gene sequences can be determined by the illumina_MiSeq_Amplicon_Deep_Sequence method. Of the gene sequences of the VHH antibody, a portion of 500 base pairs or less was set to a target region, and gene sequences included in this portion were determined. As primers used to amplify the target region, 5'gaaatacctattgcctacggc (5' side, sequence number 1) and 5'ggaaccgtagtccggaacgtc (3' side, sequence number 2) were designed. These primer sets were used in polymerase chain reaction (PCR), thereby amplifying the target region of the portion of 500 base pairs or less in the gene sequence of the VHH antibody. Next, using primer sets obtained by fusing designated sequences unique to the next-generation sequencer, that is, 5'TCGTCGGCAGCGTCAGATGTGTATAAGAGACAG- (sequence number 3) and 5'GTCTCGTGGGCTCGGAGATGTGTATAAGAGACAG- (sequence number 4) to the above-described primers, the gene sequence of the target region was determined by the illumina_MiSeq_Amplicon_Deep_Sequence method in accordance with a method recommended by the manufacturer (Illumina).

### Example 8: Translate Group of Gene Sequences of VHH Antibody Determined by Next-Generation Sequencer to Group of Amino Acid Sequences

Translation from the group of gene sequences to a group of amino acid sequences was done by commercially available analysis software (Genetyx). In the group of gene sequences and the group of amino acid sequences, sequences in which the sequence at the end completely matches a used primer, sequences in which the base length of a target region is 401 base pairs or more, sequences for which the read count is 2 or more, and sequences including a his tag sequence (6xHis) which was included in a vector sequence were selected. Furthermore, sequences in which the gene sequences are different but the amino acid sequences are identical were added together, and then the amino acid sequences were arranged in descending order of appearance frequency (read count).

### Example 9: Acquisition of Group of Amino Acid Sequences of VHH Antibody That Interacts with Her2

According to Example 8, a list of amino acid sequences of a VHH antibody that interacts with Her2 obtained in Example 5 was created. The number of types of amino acid sequences of the VHH antibody that interacts with Her2 was 18,640.

### Example 10: Acquisition of Group of Amino Acid Sequences of VHH Antibody That Binds to Her2 under Environment in Which Protein (Herceptin Monoclonal Antibody) That Interacts with Her2 Exists

According to Example 8, a list of amino acid sequences of a VHH antibody that interacts with Her2 under an environment in which a protein (Herceptin monoclonal antibody) that interacts with Her2 obtained in Example 6 exists was created. The number of types of amino acid sequences of the VHH antibody that interacts with Her2 under an environment in which a protein (Herceptin monoclonal antibody) that interacts with Her2 exists was 13,670.

### Example 11: Acquiring Group of Amino Acid Sequences (C Group) by Subtracting Group of Amino Acid Sequences (B Group) of VHH Antibody That Binds to Her2 under Environment in Which Protein (Herceptin Monoclonal Antibody) That Interacts with Her2 Exists from Group of Amino Acid Sequences (A Group) of VHH Antibody That Interacts with Her2 (C Group = A Group - B Group)

More specifically, amino acid sequences included in the following groups were selected, and the C group was acquired.

Amino acid sequences that are included in the A group (read count was 2 or more) and are not included in the B group (read count was 0) were selected (Co group = 200 types).

Among the amino acid sequences that are included in the A group (read count was 20 or more), amino acid sequences that only exists in the B group by a read count of 1/10 or less thereof (read count was 2 or more) were selected (C_{1/10} group = 360 types).

Among the amino acid sequences that are included in the A group (read count was 4 or more), amino acid sequences that only exist in the B group by a read count of 1/2 or less thereof (read count was 2 or more) were selected (C_{1/2} group = 5,000 types).

The C group was obtained as a set generated by adding the amino acid sequences included in the C₀ group, the C_{1/10} group, and the C_{1/2} group and excluding amino acid sequences that duplicated in the three groups (C group = 5,500 types).

### Example 12: Artificial Gene Synthesis of Gene Sequence That Codes Amino Acid Sequence Included in C Group

All amino acid sequences included in the C₀ group had 200 amino acid residues or less. A gene sequence that codes the amino acid sequences could artificially be synthesized as an optimized gene sequence (the optimized gene sequence here means, for example, a gene sequence that can easily be synthesized, is hardly decomposed, includes bases that are not uneven, has few repeats of sequences, and hardly takes a secondary structure) provided by a manufacturer (Eurofins). The artificially synthesized gene could be incorporated in an arbitrary expression vector.

### Example 13: Expression of VHH Antibody Protein Coded by Artificial Gene

When artificial gene incorporated in an expression vector was introduced into Escherichia coli (BL21) as a plasmid, the Escherichia coli could be caused to express a VHH antibody protein coded by the artificial gene. More specifically, the expression of an amino acid sequence coded by the artificial gene was performed by adding IPTG when the Escherichia coli was in a logarithmic growth phase. The expressed amino acid sequence (a his tag was added to the VHH antibody) was purified using a nickel immobilized resin column by a method recommended by the manufacturer (GE Healthcare).

### Example 14: Confirmation of Epitope Overlap in Cell and Tissue Staining

Fluorescent staining of cells and tissues was performed using a part of the purified VHH antibody obtained in Example 13. An anti-his tag rabbit antibody (Abcam) was used as a secondary antibody, and a red fluorescent labeled anti-IgG rabbit antibody (Thermo Fisher) was used as a tertiary antibody. Staining using a Herceptin monoclonal antibody was also performed at the same time. As a detection reagent, a green fluorescent labeled anti-IgG mouse antibody (Thermo Fisher) was used as a secondar antibody. As a sample, a tissue slice of a cancer patient, which is known to bind to a Herceptin monoclonal antibody, was used.

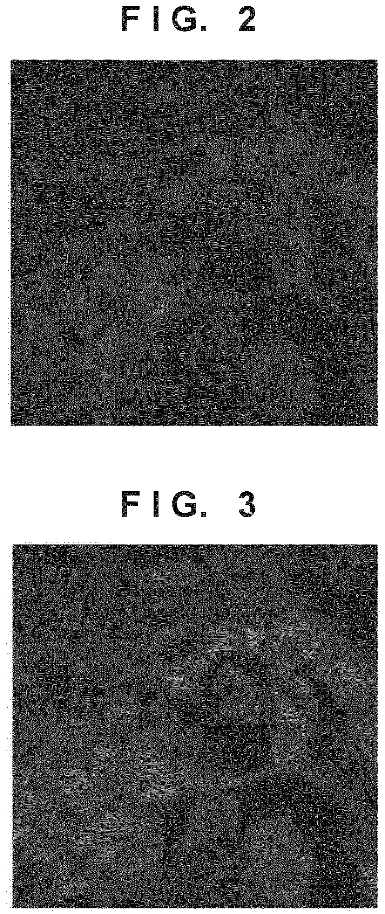

As a result, in the figure (Fig. 2) showing cell and tissue staining using the VHH antibody (sequence number 5) obtained in Example 13, it was shown that staining was successfully performed similarly to the figure (Fig. 3) showing cell and tissue staining using a control Herceptin monoclonal antibody. Hence, as is apparent, the antibody obtained by the method according to the present invention can specifically recognize an epitope to which a Herceptin monoclonal antibody binds.

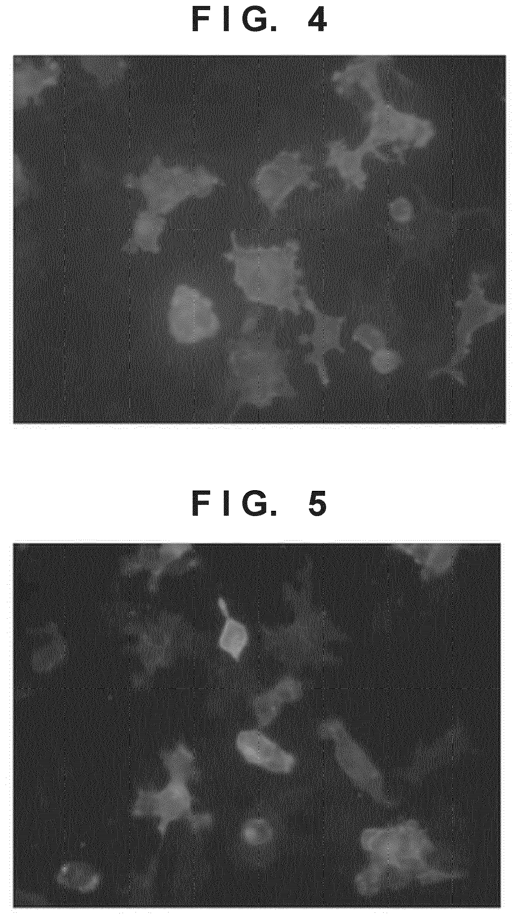

It was also confirmed that even for Her2 produced in human embryonic kidney cells (HEK 293), cell and tissue staining using by the VHH antibody obtained in Example 13 could be performed, like the Herceptin monoclonal antibody (Fig. 4: a view showing staining using the VHH antibody obtained in Example 13, Fig. 5: a view showing staining using the Herceptin monoclonal antibody).

In the above-described examples, the amino acid sequences of an antibody group for recognizing a target substance (for example, Her2) and the amino acid sequences of an antibody group for recognizing a control substance (for example, Her2 to which Herceptin binds) were determined (Examples 9 and 10). As described above, a next-generation sequencer or the like can be used for sequencing. An antibody that selectively recognizes the target substance in comparison to the control substance was specified by comparing the thus decoded amino acid sequences (Example 11). To facilitate comparison of the amino acid sequences and facilitate discovery of the antibody that selectively recognizes the target substance, antibody groups including similar amino acid sequences may be classified put into groups by clustering according to a predetermined condition, and the amino acid sequence groups may be compared between the groups, as described above.

In the comparison of the amino acid sequences, as described above, an antibody which is included in an antibody group that recognizes the target substance and whose number in an antibody group that recognizes the control substance is a predetermined threshold or less can be specified as the antibody that selectively recognizes the target substance. For example, the Co group is an antibody group that is included in the antibody group (A group) that recognizes the target substance and is not included in the antibody group (B group) that recognizes the control substance. The C₁₀ group is an antibody group which is included in the A group and whose number in the B group is 1/10 or less of the number in the A group. The C_{1/2} group is an antibody group which is included in the A group and whose number in the B group is 1/2 or less of the number in the A group.

When the combination of a target substance and a control substance that is different only in a portion that characterizes the target substance is used, an antibody that selectively recognizes the portion that characterizes the target substance can be specified, as described above. In the above-described examples, Her2 was used as the target substance, and Her2 to which Herceptin binds was used as the control substance, thereby not only simply specifying Her2 but also specifying an antibody that selectively recognizes an epitope to which Herceptin binds in the Her2 (that is, a Herceptin binding site of Her2). This point can be understood from the fact that the staining pattern of the VHH antibody obtained in Example 13 and the staining pattern of Herceptin matched (Example 14). As described above, the control substance may have a structure in which the target site of the target substance is modified, like the complex of Her2 and Herceptin used in the above-described examples (that is, Her2 whose Herceptin binding site is modified). When the target substance and the control substance are selected in this way, an antibody that selectively recognizes the target site of the target substance in comparison with other sites of the target substance can be specified. Note that as the method of modifying the target site of the target substance, a method of binding a ligand to the target substance, as described above, a method of selectively modifying the amino acid sequence in the target site of the target substance, and the like can be used.

The amino acid sequence of an antibody group that recognizes a target substance and the amino acid sequence of an antibody group that recognizes a control substance can be obtained by immunizing an animal, as described above. That is, when a target substance is given to an animal that produces an antibody, the amino acid sequence of the antibody group that is produced by the animal and recognizes the target substance can be determined. In the above-described examples, a target substance was given to an animal that produces an antibody (Example 1), and the amino acid sequence of an antibody group that recognizes the target substance in antibody groups induced in the animal was determined. As a detailed method, a gene sequence that produces the antibody group induced in the animal was determined (Example 5), phages, in which a gene having the determined gene sequence was introduced, were caused to interact with the target substance (Example 5), and an amino acid sequence corresponding to the gene sequence introduced into the phages bound to the target substance was specified as the amino acid sequence of an antibody that recognizes the target substance (Examples 7 and 8). In the above-described examples, similarly, the amino acid sequences of an antibody group that recognizes the control substance in antibody groups induced in the animal were determined. As a detailed method, phages, in which a gene having a determined gene sequence was introduced, were caused to interact with the control substance (Example 6), and an amino acid sequence corresponding to the gene sequence introduced into the phages bound to the control substance was specified as the amino acid sequence of an antibody that recognizes the control substance (Examples 7 and 9). Note that in the above-described examples, the gene sequence of a gene of an antibody, the gene being included in lymphocytes obtained from an animal, was determined, thereby sequencing the group of genes that produce the antibody group induced in the animal (Example 5).

As shown in Example 13, when the amino acid sequence of the antibody that selectively recognizes the target substance in comparison with the control substance is specified, an antibody having the specified amino acid sequence can be manufactured.

### INDUSTRIAL APPLICABILITY

By the method of the present invention, it is possible to easily and efficiently acquire an antibody (group) that specifically binds to a target substance, and the present invention can suitably be used for, for example, an antibody for a specific epitope, a nucleic acid that codes the antibody, a detection kit, a detection method, and a treatment research of a specific disease.

The present invention is not limited to the above embodiments and various changes and modifications can be made within the spirit and scope of the present invention. Therefore, to apprise the public of the scope of the present invention, the following claims are made.

This application claims priority from Japanese Patent Application No. 2018-103024 filed May 30, 2018, which is hereby incorporated by reference herein.

## Claims

1. A method of acquiring an antibody that specifically recognizes a target substance, **characterized by** subtracting an antibody group that recognizes a control substance from an antibody group that recognizes the target substance.

2. The method according to claim 1, wherein the antibody is a VHH antibody.

3. The method according to claim 1 or 2, wherein the subtraction detects a difference in a result of a next-generation sequencer.

4. The method according to any one of claims 1 to 3, wherein the antibody is used for classification of a mammalian cell.

5. The method according to any one of claims 1 to 3, wherein the antibody is used for classification of a class of a living thing.

6. The method according to any one of claims 1 to 3, wherein the antibody is used for classification of a virus.

7. The method according to any one of claims 1 to 3, wherein the antibody is used for classification of a molecule.

8. The method according to any one of claims 1 to 7, wherein the subtracting the antibody group that recognizes the control substance from the antibody group that recognizes the target substance includes comparing amino acid sequences of the antibody group that recognizes the target substance with amino acid sequences of the antibody group that recognizes the control substance.

9. The method according to any one of claims 1 to 8, wherein the antibody that specifically recognizes the target substance is included in the antibody group that recognizes the target substance, and
the number of antibodies, in the antibody group that recognizes the control substance, that specifically recognize the target substance is not more than a predetermined threshold.

10. An antibody acquired by a method of any one of claims 1 to 9.

11. The antibody according to claim 10, wherein the antibody is a peptide.

12. An antibody specifying method comprising a step of specifying an antibody that selectively recognizes a target substance in comparison to a control substance by comparing amino acid sequences of an antibody group that recognizes the target substance with amino acid sequences of an antibody group that recognizes the control substance.

13. The specifying method according to claim 12, wherein the antibody specified in the step of specifying is included in the antibody group that recognizes the target substance, and
the number of antibodies, specified in the step of specifying, in the antibody group that recognizes the control substance is not more than a predetermined threshold.

14. The specifying method according to claim 12 or 13, wherein the control substance has a structure in which a target site of the target substance is modified, and
the antibody specified in the step of specifying selectively recognizes the target site of the target substance in comparison to other sites of the target substance.

15. The specifying method according to any one of claims 12 to 14, further comprising steps of:
giving the target substance to an animal that produces the antibody;
determining the amino acid sequences of the antibody group that recognizes the target substance among antibody groups induced in the animal; and
determining the amino acid sequences of the antibody group that recognizes the control substance among the antibody groups induced in the animal.

16. The specifying method according to claim 15, further comprising a step of determining gene sequences that produce the antibody group induced in the animal,
wherein the step of determining the amino acid sequences of the antibody group that recognizes the target substance includes steps of:
causing a phage, in which a gene having the determined gene sequence is introduced, to interact with the target substance; and
specifying an amino acid sequence corresponding to the gene sequence introduced into the phage bound to the target substance as the amino acid sequence of the antibody that recognizes the target substance, and
the step of determining the amino acid sequences of the antibody group that recognizes the control substance includes steps of:
causing a phage, in which a gene having the determined gene sequence is introduced, to interact with the control substance; and
specifying an amino acid sequence corresponding to the gene sequence introduced into the phage bound to the control substance as the amino acid sequence of the antibody that recognizes the control substance.

17. The specifying method according to claim 15 or 16, wherein the step of determining the gene sequences of the genes that produce the antibody group induced in the animal includes determining a gene sequence of a gene of an antibody, the gene being included in a lymphocyte obtained from the animal.

18. The specifying method according to any one of claims 15 to 17, wherein the antibody is a VHH antibody, and the animal is an alpaca.

19. An antibody manufacturing method **characterized by** comprising steps of:
specifying an amino acid sequence of an antibody that selectively recognizes a target substance in comparison to a control substance in accordance with a specifying method of any one of claims 12 to 18; and
manufacturing the antibody including the specified amino acid sequence.

20. An antibody manufactured in accordance with a manufacturing method of claim 19.


SEQUENCE LISTING

<110> COGNANO, Inc.

<120> Method for obtaining antibody, identifying antibody, and manufacturing
antibody, and antibody

<130> P219-0591WO

<160> 5

<170> PatentIn version 3.1

<210> 1
<211> 21
<212> DNA
<213> Artificial Sequence

<220>
<223> Primer Sequence

<400> 1
gaaataccta ttgcctacgg c                                                21


<210> 2
<211> 21
<212> DNA
<213> Artificial Sequence

<220>
<223> Primer Sequence

<400> 2
ggaaccgtag tccggaacgt c                                                21


<210> 3
<211> 33
<212> DNA
<213> Artificial Sequence

<220>
<223> Primer Sequence

<400> 3
tcgtcggcag cgtcagatgt gtataagaga cag                                   33


<210> 4
<211> 34
<212> DNA
<213> Artificial Sequence

<220>
<223> Primer Sequence

<400> 4
gtctcgtggg ctcggagatg tgtataagag acag                                  34

<210> 5
<211> 129
<212> PRT

<213> Vicugna pacos

<400> 5
Met Ala Gln Val Gln Leu Gln Glu Ser Gly Gly Gly Leu Val Gln Ala
1               5                   10                  15
Gly Gly Ser Leu Arg Leu Ser Cys Ala Ala Ser Gly Arg Thr Leu Ser
            20                  25                  30
Gly Ala Ala Met Gly Trp Phe Arg Gln Ala Pro Gly Lys Glu Arg Glu
        35                  40                  45
Phe Val Ala Gly Ile Ser Trp Ser Thr Ser Arg Thr Gln Tyr Ala Asp
    50                  55                  60
Ser Val Lys Gly Arg Phe Thr Ile Ser Arg Asp Asn Ala Lys Asn Thr
65                  70                  75                  80
Val Phe Leu Gln Met Asn Ser Leu Asn Ala Glu Asp Thr Ala Val Tyr
                85                  90                  95
Tyr Cys Ala Ala Asp Gly Arg Phe Tyr Ser Asp Tyr Val Trp Ser Ser
            100                 105                 110
Pro Asp Glu Tyr Ala Tyr Trp Gly Gln Gly Thr Gln Val Thr Val Ser
        115                 120                 125
Ser